(19) Europäisches Patentamt
European Patent Office
Office européen des brevets

(11) Numéro de publication: **0 102 297**
**B1**

# (12) FASCICULE DE BREVET EUROPEEN

(45) Date de publication du fascicule du brevet: **23.10.85**

(51) Int. Cl.[4]: **C 07 C 65/34**, C 07 C 51/487, C 07 C 51/44

(21) Numéro de dépôt: **83401694.1**

(22) Date de dépôt: **23.08.83**

(54) **Procédé de décomposition d'un complexe d'acide ortho-benzoyl-benzoique, de fluorure d'hydrogène et de trifluorure de bore.**

(30) Priorité: **01.09.82 FR 8214920**

(43) Date de publication de la demande:
**07.03.84 Bulletin 84/10**

(45) Mention de la délivrance du brevet:
**23.10.85 Bulletin 85/43**

(84) Etats contractants désignés:
**BE CH DE FR GB IT LI NL SE**

(56) Documents cités:
**FR - A - 814 838**
**GB - A - 486 886**
**GB - A - 501 670**

(73) Titulaire: **ATOCHEM, 12/16, allée des Vosges, F-92400 Courbevoie (FR)**

(72) Inventeur: **Devic, Michel, 134, rue Edmond Locard, F-69005 Lyon (FR)**

EP 0 102 297 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de décomposition de complexes formés d'acide orthobenzoyl-benzoïque (acide OBB), éventuellement substitué, d'acide fluorhydrique (HF) et de trifluorure de bore ($BF_3$), de formule générale:

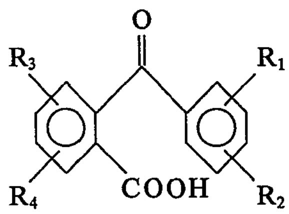 nHF, $mBF_3$ (I)

dans laquelle $R_1$, $R_2$, $R_3$, $R_4$, représentent un atome d'hydrogène ou d'halogène, ou un groupe alkyle linéaire ou ramifié, possédant 1 à 5 atoms de carbone, n et m étant compris entre 1 et 6.

De tels complexes peuvent être obtenus selon la demande de brevet français n° 2 496 097 au nom de la PCUK.

Pour isoler l'acide O.B.B. à partir de tels complexes, les techniques décrites dans le brevet français n° 814 838 et dans le brevet britannique n° 486 886 ne peuvent convenir.

Elles ne peuvent en effet permettre en particulier la séparation d'un acide organique du $BF_3$ initialement combiné à lui sous forme d'une composition complexe qu'à la condition qu'au cours du processus distillatoire qui les caractérise, une telle composition puisse fournir différentes compositions dérivées bouillant à température constante, ce qui n'est aucunement le cas des compositions complexes auxquelles se rapportent l'invention.

Afin de pouvoir isoler l'acide OBB, les complexes doivent subir un traitement par de l'eau bouillante ou par de la soude diluée, qui détruit HF et $BF_3$. La récupération du catalyseur HF, $BF_3$ de synthèse de l'anthraquinone décrite dans la demande de brevet de la PCUK citée plus haut, ne peut pas être réalisée d'une manière économiquement viable.

Un chauffage prolongé du complexe à 150—200°C permet de le décomposer mais entraîne simultanément la dégradation presque totale de l'acide OBB lui-même. L'eau formée lors de cette dégradation de l'acide OBB se combine de plus à HF et $BF_3$ ce qui rend le catalyseur impropre à un recyclage.

Un chauffage prolongé sous vide du complexe à une température plus basse, 50 à 100°C, permet de limiter la décomposition de l'acide OBB, mais laisse subsister une partie du catalyseur dans l'acide OBB, ce qui rend le procédé non économique.

Le chauffage du complexe à faible température dans un solvant inerte ne permet pas non plus de récupérer la totalité du catalyseur et provoque une dégradation encore importante de l'acide OBB.

Le procédé selon l'invention permet de pallier les inconvéniente que présentent de telles méthodes, et de récupérer pratiquement quantitativement HF et $BF_3$ initialement complexés avec l'acide OBB, sans dégrader ce dernier.

Le procédé selon l'invention consiste plus particulièrement à soumettre le complexe de formule générale (I) à l'action d'un solvant inerte vis-à-vis du complexe, de HF et de $BF_3$, dans une colonne à distiller fonctionnant avec reflux de solvant très supérieur au débit d'alimentation, et ne provoquant pas de rétention de la phase liquide.

Comme solvants appropriés à la mise en oeuvre du procédé selon l'invention on peut citer les hydrocarbures aliphatiques halogénés, en particulier le chlorure de méthylène et le dichloro-1,2-éthane, et les hydrocarbures aromatiques fluorés.

Le but de l'invention est d'autant mieux atteint que le solvant approprié choisi est bon solvant de l'acide OBB et mauvais solvant de HF.

Le procédé selon l'invention consiste à opérer dans une colonne à distiller ayant un nombre de plateaux supérieur à 8, au mieux compris entre 15 et 40, présentant un volume de rétention minimum de la phase liquide: garnissage métallique du type MULTIKNIT (marque déposée de la Société TISSMETAL), ou formé de ressorts ou d'anneaux en acier inoxydables par exemple, et fonctionnant avec un reflux de solvant, donc un débit de vapeur de solvant, suffisant pour assurer la dissociation du complexe en HF et $BF_3$ d'une part, en acide OBB d'autre part et pour vaporiser HF, soit 5 à 40 fois et au mieux 10 à 20 fois en poids le débit d'introduction dans la colonne, le niveau d'introduction étant avantageusement situé dans le premier tiers supérieur de la colonne dont la température est comprise entre 20°C, point d'ébullition de HF anhydre, et 150°C et dont la pression, inférieure ou supérieure (2 à 15 bar) à la pression atmosphérique et au mieux égale ou légèrement supérieure à celle-ci, est réglée pour rester dans cette plage de température.

L'acide OBB, substitué ou non, récupéré à partir du bouilleur de la colonne peut être soumis à un lavage subséquent avec de l'eau, additionnée ou non d'un hydroxyde alcalin, pour éliminer les traces de $BF_3$ et d'HF.

Le procédé selon l'invention peut être réalisé en continu ou en discontinu.

La figure 1 montre un schéma de fonctionnement d'un mode de réalisation de l'invention: le complexe acide OBB, HF, $BF_3$ en solution dans HF est introduit dans la colonne 1 par le conduit 2, tandis que le solvant est porté à ébullition dans le bouilleur 3. Les vapeurs de solvant et d'HF sont condensées dans le condenseur 4 et le $BF_3$ gazeux sort par le conduit 5. HF, séparé du solvant dans le décanteur 6 sort par le conduit 7 et le solvant est réintroduit en tête de colonne par le conduit 8. Un soutirage 9 permet d'éliminer les faibles quantités d'impuretés telles que les hydrates de $BF_3$ et de HF pouvant s'accumule dans

le décanteur 6.

L'acide OBB en solution dans du solvant est soutiré par le conduit 10 et lavé par de l'eau en 11. Le solvant, évaporé dans l'évaporateur 12 est réintroduit dans la colonne par le conduit 13 et l'acide OBB fondu sort par le conduit 14.

Le schéma de la figure 2 diffère du schéma 1 par le mode de séparation de HF du solvant. Les vapeurs de solvant et de HF sont condensées dans le condenseur 4 et le $BF_3$ gazeux s'échappe par le conduit 5. Le solvant et HF liquide sont introduits dans la colonne 6 d'où HF gazeux est éliminé par le conduit 7. Le solvant retiré en pied de la colonne 6 est réintroduit dans la colonne de décomposition 1 par le conduit 8.

Il est possible encore d'opérer la décomposition du complexe acide OBB, HF, $BF_3$ en deux stades, par exemple en alimentant dans une seconde colonne, fonctionnant sous une pression de 2 à 15 bars et à une température de 60° à 150°C, le complexe partiellement décomposé provenant d'une première colonne fonctionnant à pression atmosphérique ou sous vide partiel à une température comprise entre 40 et 60°C.

Les exemples suivants, non limitatifs, illustrent la présente invention. Les quantités ou concentrations d'acide OBB sont déterminées analytiquement par chromatographie liquide.

Exemple 1

On prépare le complexe acide OBB, HF, $BF_3$ en solution dans HF en faisant réagir en proportion, selon la demande de brevet français n° 2 496 097, 1 mole d'anhydride phtalique et 1 mole de benzène dans 10 moles de HF et 10 moles de $BF_3$. Après dégazage à —40°C d'une partie du $BF_3$ on obtient pour 1 mole d'anhydride phtalique engagée, une solution de complexe de l'acide OBB dans HF contenant 20 3,3 g d'acide OBB, 306,7 g de $BF_3$, 200 g d'HF total et 22,7 g d'impuretés de la réaction.

Dans une colonne en acier inoxydable de 38 mm de diamètre intérieur et contenant, sur une hauteur de 1,20 m un garnissage MULTIKNIT en acier inoxydable 18/10 Mo de porosité 93,3% et de surface 215 $dm^2/dm^3$, on établit un reflux de chlorure de méthylène, la température de la colonne étant de 41°C e la pression, la pression atmosphérique. Le débit de vaporisation du chlorure de méthylène dans le bouilleur est de 3,87 kg/h.

On introduit alors en 28 minutes, 110 g de la solution de complexe. On maintient ensuite le reflux pendant 20 minutes, puis on évapore le chlorure de méthylène contenu dans le bouilleur de la colonne. Durant l'opération le $BF_3$ gazeux et l'HF sont recueillis en tête de colonne. 0,5 g de résidu contenant de l'hydrate de $BF_3$ sont recueillis dans le décanteur placé en tête de colonne.

On recueille en fin d'opération 33,3 g d'acide OBB brut contenant 28,6 g d'acide OBB, soit un rendement de récupération de l'acide OBB de 93,8%.

L'acide OBB brut contient 0,43 g de $BF_3$ et 0,1 g d'HF. Le taux d'efficacité de la décomplexation est de 99% pour le $BF_3$ et de 99,6% pour l'HF.

Exemple 2

On opère dans la même colonne et dans les mêmes conditions de température et de pression dans la colonne que dans l'exemple 1.

Le débit de vaporisation du chlorure de méthylène est de 2,7 kg/h.

On introduit en 42 minutes 94,6 g de la solution de complexe préparée selon l'exemple 1.

On maintient ensuite le reflux durant 30 minutes. La solution recueillie dans le bouilleur en fin d'opération est lavée après refroidissement par 475 ml d'eau distillée. La solution d'acide OBB dans le chlorure de méthylène ainsi lavée est évaporée et on obtient 29,6 g d'acide OBB brut contenant 26 g d'OBB soit un rendement de récupération de l'acide OBB de 99%.

L'acide OBB brut est exempt d'HF et de $BF_3$. Le taux d'efficacité de la décomplexation est de 99% pour le $BF_3$ et de 99,2% pour l'HF comptetenu du $BF_3$ et de l'HF récupérés dans l'eau de lavage de la solution d'acide OBB brut dans le chlorure de méthylène.

Exemple 3

On opère dans la même colonne que pour l'exemple 2, à pression atmosphérique, mais à 84°C et en présence de dichloro-1,2-éthane au lieu de chlorure de méthylène. Le débit de vaporisation du dichloroéthane est de 4,22 kg/h. On introduit en 22 minutes 110 g de la solution du complexe préparée selon l'exemple 1. Le reflux de solvant est ensuite maintenu durant 20 minutes avant évaporation du dichloroéthane contenu dans le bouilleur.

On recueille 32 g d'acide OBB brut contenant 28,0 g d'acide OBB soit un rendement de récupération de l'acide OBB de 92%.

L'acide OBB brut contient 0,33 g de $BF_3$ et 0,1 g d'HF soit un taux d'efficacité de la décomplexation de 99,3% pour le $BF_3$ et de 99,6% pour l'HF.

Exemple 4

On prépare un complexe de l'acide ortho (éthyl-4-benzoyl) benzoïque (OEBB) en faisant réagir selon la demande de brevet français n° 2 496 097, 26,6 g d'anhydride phtalique avec 19,07 g d'éthylbenzène dans 35,9 g d'HF et 121,77 g de $BF_3$ pendant 30 minutes à —40°C. Après réaction et dégazage à —40°C on obtient 144,4 g d'une solution du complexe dans HF qui est injectée en 20 minutes dans la colonne décrite dans l'exemple 1 et travaillant dans les

mêmes conditions de température et de pression, avec aussi le chlorure de méthylène.

Le débit de reflux du chlorure de méthylène est de 3,8 kg/h. Après la fin de l'introduction de la solution du cmplexe le reflux de solvant est maintenu durant 20 minutes avant évaporation du solvant contenu dans le bouilleur.

On recueille 43,31 g d'acide OEBB brut contenant 39,07 g d'acide OEBB et les taux de récupération de $BF_3$ et d'HF sont respectivement de 99,3% et de 99,6%.

## Revendications

1. Procédé de décomposition des complexes d'acides orthobenzoylbenzoïques, fluorure d'hydrogène, trifluorure de bore, de formule générale:

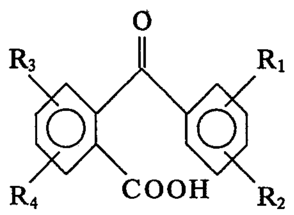

nHF, $mBF_3$

dans laquelle $R_1$, $R_2$, $R_3$, $R_4$, représentent un atome d'hydrogène ou d'halogène ou un groupe alkyle linéaire ou ramifié possédant 1 à 5 atomes de carbone, n et m étant compris entre 1 et 6, pour obtenir l'acide orthobenzoylbenzoïque d'une part, le fluorure d'hydrogène et le trifluorure de bore d'autre part, caractérisé en ce que le complexe est soumis à l'action d'un solvant inerte à une température au moins égale à 20° C, dans une colonne à distiller ayant un nombre de plateaux théoriques au moins égal à 8, et fonctionnant avec reflux de solvant égal à 5 à 40 fois, en poids, le débit d'alimentation de la colonne, et l'on soutire l'acide en solution dans le solvant du bouilleur de cette colonne.

2. Procédé selon la revendication 1 caractérisé en ce que la colonne comporte 15 à 40 plateaux théoriques.

3. Procédé selon l'une des revendications 1 ou 2 caractérisé en ce que le débit de reflux de solvant est égal à 10 à 20 fois, en poids, le débit d'alimentation de la colonne.

4. Procédé selon l'une quelconque des revendications 1 à 3 caractérisé en ce que la température de la colonne est comprise entre 20 et 150° C.

5. Procédé selon la revendication 1 caractérisé en ce que le solvant est un hydrocarbure aliphatique halogéné.

6. Procédé selon la revendication 5 caractérisé en ce que le solvant est le chlorure de méthylène.

7. Procédé selon la revendication 5 caractérisé en ce que le solvant est le dichloro-1,2-éthane.

8. Procédé selon la revendication 1, caractérisé en ce que le solvant est un hydrocarbure aromatique fluoré.

## Patentansprüche

1. Verfahren zur Zersetzung von Komplexen von Orthobenzoylbenzoesäuren mit Fluorwasserstoff und Bortrifluorid der allgemeinen Formel:

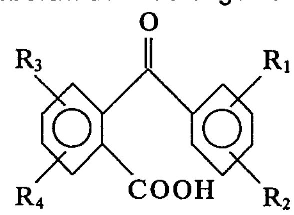

nHF, $mBF_3$

in der $R_1$, $R_2$, $R_3$, $R_4$ ein Wasserstoff- oder Halogenatom oder eine lineare oder verzweigte Alkylgruppe mit 1 bis 5 Kohlenstoffatomen bedeuten und n und m Werte von 1 bis 6 besitzen, zur Herstellung der Orthobenzoylbenzoesäure einerseits und des Fluorwasserstoffs und des Bortrifluorids andererseits, dadurch gekennzeichnet, daß der Komplex in einer Destillationskolonne mit mindestens 8 theoretischen Böden bei einer Temperatur von mindestens 20° C der Einwirkung eines inerten Lösungsmittels unterworfen wird, wobei die Kolonne bei einem Lösungsmittelrückfluß der 5- bis 40fachen Gewichtsmenge der in die Kolonne eingesetzten Menge betrieben wird, und daß man die Säure in dem Lösungsmittel gelöst aus dem Kocher dieser Kolonne abzieht.

2. Verfahren nach Anspruch 1, dadurch gekennzeichnet, daß die Kolonne 15 bis 40 theoretische Böden besitzt.

3. Verfahren nach Anspruch 1 oder 2, dadurch gekennzeichnet, daß die Rückflußmenge des Lösungsmittels der 10- bis 20fachen Gewichtsmenge der in die Kolonne eingesetzten Menge entspricht.

4. Verfahren nach einem der Ansprüche 1 bis 3, dadurch gekennzeichnet, daß die Kolonnentemperatur zwischen 20 und 150° C liegt.

5. Verfahren nach Anspruch 1, dadurch gekennzeichnet, daß das Lösungsmittel ein halogenierter aliphatischer Kohlenwasserstoff ist.

6. Verfahren nach Anspruch 5, dadurch gekennzeichnet, daß das Lösungsmittel Methylenchlorid ist.

7. Verfahren nach Anspruch 5, dadurch gekennzeichnet, daß das Lösungsmittel 1,2-Dichlorethan ist.

8. Verfahren nach Anspruch 1, dadurch gekennzeichnet, daß das Lösungsmittel ein fluorierter aromatischer Kohlenwasserstoff ist.

## Claims

1. Process for decomposition of complexes of ortho-benzoylbenzoic acids with hydrogen fluoride and boron trifluoride, of general formula:

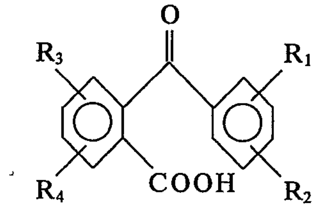

nHF, $mBF_3$

in which $R_1$, $R_2$, $R_3$ and $R_4$ denote a hydrogen or halogen atom or a linear or branched alkyl group possessing 1 to 5 carbon atoms, n and m being between 1 and 6, to obtain the ortho-benzoylbenzoic acid on the one hand, and hydrogen fluoride and boron trifluoride on the other hand, characterised in that the complex is subjected to the action of an inert solvent at a temperature equal to at least 20° C, in a distillation column having a number of theoretical plates equal to at least 8 and operating with solvent reflux equal to 5 to 40 times, by weight, the column feed rate, and the acid dissolved in the solvent is drawn off from the boiler of this column.

2. Process according to Claim 1, characterised in that the column contains 15 to 40 theoretical plates.

3. Process according to one of Claims 1 or 2, characterised in that the rate of solvent reflux is equal to 10 to 20 times, by weight, the column feed rate.

4. Process according to any one of Claims 1 to 3, characterised in that the temperature of the column is between 20 and 150° C.

5. Process according to Claim 1, characterised in that the solvent is a halogenated aliphatic hydrocarbon.

6. Process according to Claim 5, characterised in that the solvent is methylene chloride.

7. Process according to Claim 5, characterised in that the solvent is 1,2-dichloroethane.

8. Process according to Claim 1, characterised in that the solvent is a fluorinated aromatic hydrocarbon.

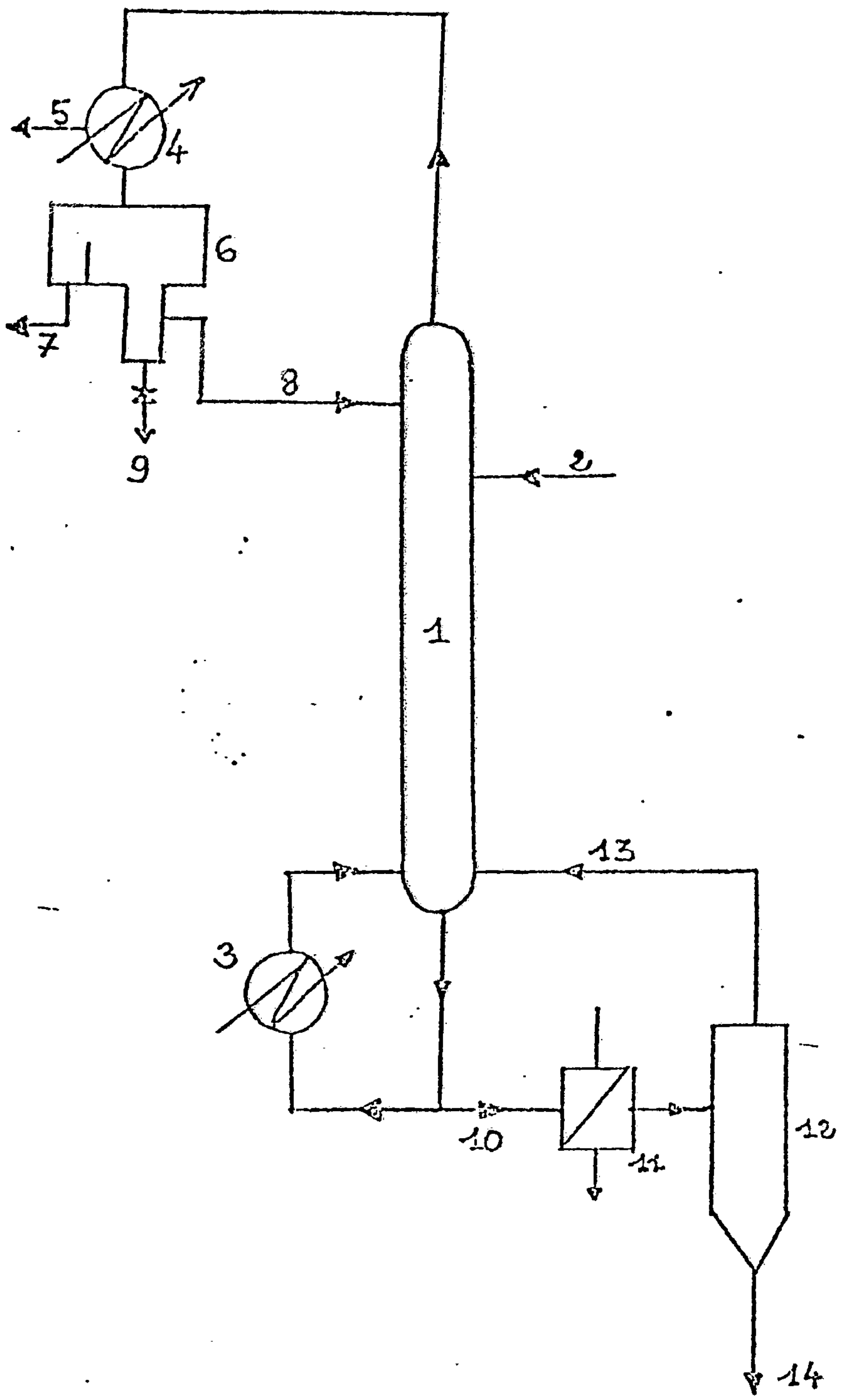


FIG. 1

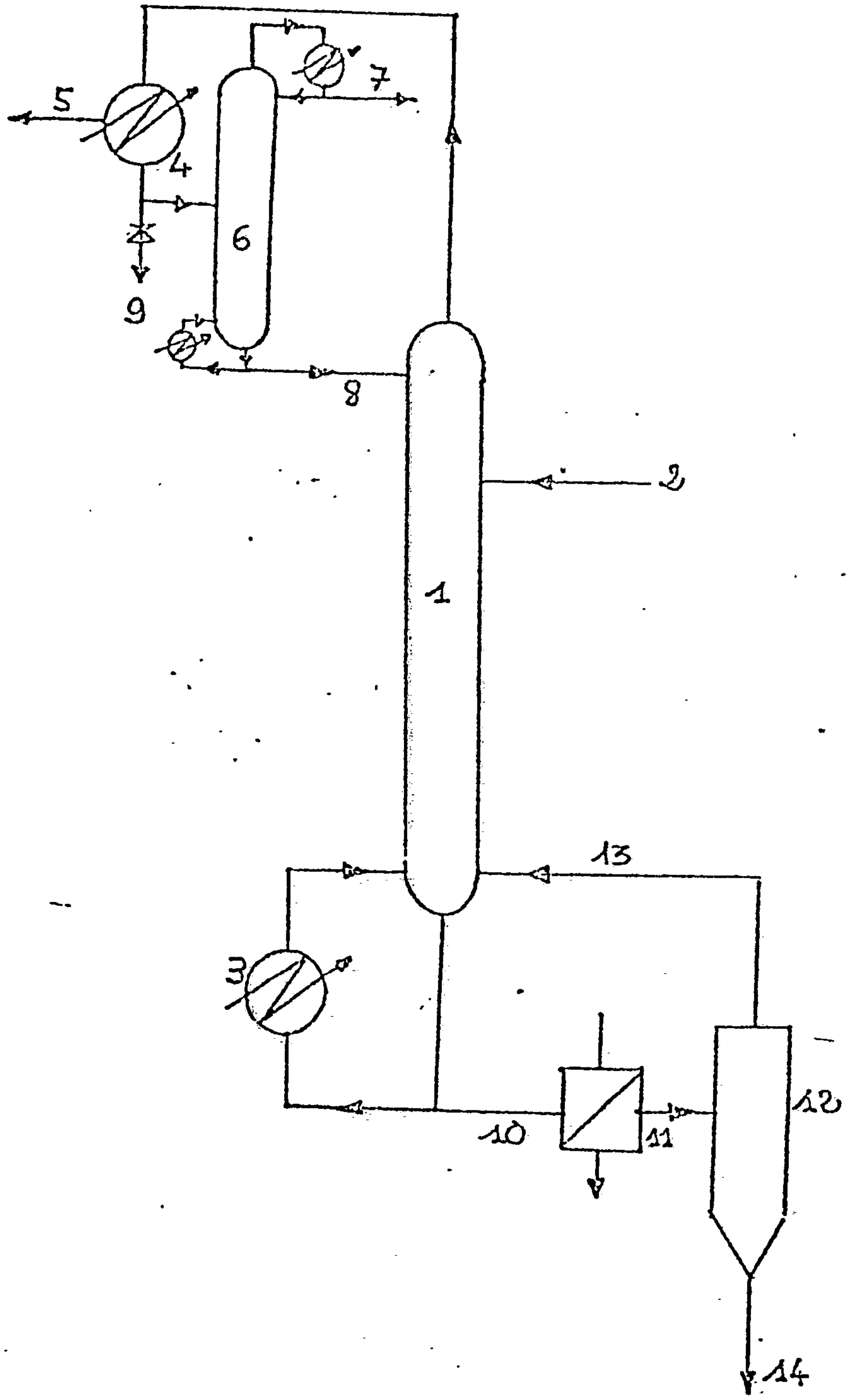


FIG. 2